# EUROPEAN PATENT APPLICATION

(11) **EP 2 486 976 A1**
(43) Date of publication of application: **15.08.2012**
(21) Application number: 12167131.7
(22) Date of filing: 08.09.2005
(51) Int. Cl.: B01J 25/00, B01J 25/02, B01J 37/025, C07B 31/00, C07C 29/17, C07C 29/141, C07C 209/48

(54) **The production and use of supported activated base metal catalysts for organic transformation**

(62) Divisional of application: 05786365.6
(71) Applicant: Evonik Degussa GmbH, 45128 Essen (DE)
(72) Inventor: Ostgard, Daniel, Dr., 63801 Kleinostheim (DE); Röder, Stefan, 36391 Sinntal (DE); Berweiler, Monika, 63477 Maintal (DE); Quandt, Thomas, Dr., 45772 Marl (DE)

(57) **Abstract**

Method for the production of supported activated metal catalysts, whereby an alloy, a metal powder, a pore builder is dispersed in a water, the dispersion is sprayed on a support which is the dried, calcined and activated.

The catalysts can be used for organic transformations, i.e. for hydrogenation reactions.

## Description

The present invention relates to the production and the use of supported activated base metal catalysts.

Activated metal catalysts are known in the field of chemical engineering as Raney-type catalysts. They are used, largely in powder form, for a large number of hydrogenation, isomerization, hydration, hydrogenolysis, reductive amination, reductive alklyation, dehydration, oxidation, dehydrogenation, rearrangement and other reactions of organic compounds, known collectively here as catalytic transformations of organic compounds.

These powdered catalysts are prepared from an alloy of a catalytically-active metal, also referred to herein as a catalyst metal, with a further alloying component which is soluble in alkalis. Mainly nickel, cobalt, copper, or iron are used as catalyst metals. Aluminum is generally used as the alloying component which is soluble in alkalis, but other components may also be used, in particular zinc and silicon or mixtures of these with aluminum.

These alloys may optionally contain one or more promoter elements before they are leached, or one or more promoters may be adsorbed onto the activated catalyst after it has been leached. In some cases it is also advantageous to have one or more promoters in the alloy prior to activation and to add one or more promoters to the activated promoted catalyst after it has been leached. In this situation, the one or more promoters that were added to the alloy may be the same or may be different than the one or more promoters that were added to the activated catalyst after leaching. Suitable promoter elements include those from the IA, IIA, IIIB, IVB, VB, VIB, VIIB, VIII, IB, IIB IIIA, IVA, VA, VIA, the Lanthanide and the actinide families of elements from the periodic table of elements.

These so-called Raney alloys are generally prepared by the ingot casting process. In that process a mixture of the catalyst metal and, for example, aluminum are first melted and casted into ingots. Typical alloy batches on a production scale amount to about ten to one hundred kg per ingot.

According to DE 21 59 736 cooling times of up to two hours were obtained. This corresponds to an average rate of cooling of about 0. 2 /s. In contrast to this, rates of 10² to 10⁶ K/s are achieved in processes where rapid cooling is applied (for example an atomizing process). The rate of cooling is affected in particular by the particle size and the cooling medium (see Materials Science and Technology edited by R. W. Chan, P. Haasen, E. J. Kramer, Vol. 15, Processing of Metals and Alloys, 1991, VCH-Verlag Weinheim, pages 57 to 110). A process of this type is used in EP 0 437 788 B 1 in order to prepare a Raney alloy powder. In that process the molten alloy at a temperature of 50 to 500 °C above its melting point is atomized and cooled using water and/or a gas. It is also possible to produce rapidly cooled alloys where the molten alloy is added dropwise to a liquid coolant, such as water, to form rapidly cooled granules. These rapidly cooled granules may be ground for use in the production of the catalysts described here and/or the particle size distribution of the rapidly cooled alloy could be controlled via its addition rate into the cooling medium.

To prepare a powder Raney-type catalyst, the Raney alloy is first finely milled if it has not been produced in the desired powder form during preparation. Then the aluminum is partly (and if need be, totally) removed by extraction with alkalis such as, for example, caustic soda solution to activate the alloy powder. In general, the appropriate metal hydroxides from the groups Ia and IIa of the periodic table and their mixtures with each other and with additional metal hydroxides may be used for this leaching process. In some cases, acids may also be used for the extraction of the soluble component in these raney-type alloys. Following extraction of the aluminum, the remaining power has a high specific surface area (BET), between 5 and 100 m²/g, and is rich in active hydrogen. The activated catalyst powder is pyrophoric and stored under water or organic solvents or is embedded in organic compounds which are preferably but are not necessarily solid at room temperature.

Powdered catalysts have the disadvantage that they can be used only in a batch process and, after the catalytic reaction, have to be separated from the reaction medium by costly sedimentation and/or filtration. Therefore a variety of processes for preparing moulded items which lead to activated metal fixed-bed catalysts after extraction of the aluminum have been disclosed. Thus, for example, coarse particulate Raney alloys, i.e., Raney alloys which have only been coarsely milled, are obtainable and these can be activated by a treatment with caustic soda solution. Extraction and consequently activation then occur only in a surface layer the thickness of which can be adjusted by the conditions used during extraction.

Substantial disadvantages of catalysts prepared by these prior methods are their poor mechanical stability of the activated outer layer, their low level of porosity, and their relatively very low percentage of activated metal. Since only the outer layer of these catalysts is catalytically active, abrasion leads to rapid deactivation and renewed activation of the deeper lying layers of alloy with caustic soda solution only leads, at best, to partial reactivation. Perhaps the most important disadvantage of surface activated coarse fixed bed particles of Raney-type alloys is their high concentration of unactivated alloy that can present itself as a safety risk. This unactivated Raney-type alloy is not inert and it can react under certain reaction conditions (e.g., high temperatures in the presence of water, acidic compounds or basic compounds) to produce aluminium based compounds and a considerable volume of hydrogen. Although there may be hydrogen present during the reaction, the safety danger involved with the uncontrollable production of a large volume of a gas, let alone a highly flammable gas such as hydrogen, in a confined reactor space is without question the most serious disadvantage of this catalyst. This uncontrollable evolution of hydrogen in a reactor could lead to an explosion and the consequential undesired release of chemicals into the environment.

The European Patent EP 0 648 534 B1 describes shaped, activated Raney metal fixed-bed catalysts and their preparation. To avoid the disadvantages described above, e.g., poor mechanical stability resulting from activating the outer layer of the particle, these catalysts were stabilized with the appropriate amount of binder. These catalysts were prepared by forming a homogeneous mixture of at least one catalyst alloy powder, pore builders, and a binder. The catalyst alloys each contain at least one catalytically active catalytic metal and an extractable alloying component. The pure catalyst metals or mixtures thereof which do not contain extractable components can be used as binders. The use of binder material in an amount of 0. 5 to 20 weight percent with respect to the catalyst alloy, is essential in order to achieve sufficient mechanical stability after activation. After shaping the catalyst alloy and the binder with conventional shaping aids and pore producers, the freshly prepared items which are obtained are calcined at temperatures below 850 °C. As a result of sintering processes in the finely divided binder, this produces solid compounds between the individual particles of the catalysts alloy. These compounds, in contrast to catalyst alloys, are non-extractable or only extractable to a small extent so that a mechanically stable structure is obtained even after activation. Although this technology is preferred over the use of surface activated coarse alloy grains, it still has the disadvantage of containing a large amount of unactivated, and consequently unused, alloy in the center of the catalytic body. Even if these tablets are thoroughly activated, the active sites in the center of these formed bodies are clearly under the influence of mass transfer limitation and as such, they are of less value. With the current invention, not only is all of the costly alloy on the surface, but all of the active sites of the correspondingly leached catalyst are also located in the outer shell of the formed body where they will experience little if any mass transfer limitations. As taught in European patent EP 984831 B1, rapidly cooled alloys with very fine phase structures can be used to produce formed bodies without metallic binders and such technology may also be applicable to the current invention. The current invention covers the use of binders and any technologies (i.e., the use of rapidly-cooled alloys with dendritic phase domains) that may not require binders for the stabilization of this catalyst.

Another version of this shell activated technology involves the production of alloy granules via their agglomeration in appropriately equipped mixers under the right conditions. The advantages of the granules is that they too exhibit high activities and selectivities at satisfactory bulk densities while being relatively easy to produce. The activated base metal granules were made by agglomerating particles of the desired alloys along with organic and/or inorganic binders, calcining these granules, and activating only their outer shells in caustic solution in order to make them catalytically active (W02005042153).

Depending on the conditions of catalyst preparation and the catalyst alloys being used, the use of both organic and inorganic binders may not be necessary. These granules are formed by mixing the desired alloy powder(s) with organic and/or inorganic binders and water in such a way that the particles agglomerate into granules. These granules can be made by mixing the above mentioned mixture between two parallel plates (plate granulator), or in any other suitable pieces of high energy mixers (e.g., Eirich or Lödige mixers). After mixing, the granules are dried, calcined, activated in caustic, and washed. The organic binder can be chosen such that it expands or "foams up" during either mixing, drying or calcination thereby producing a metallic foam structure that exhibits a high porosity while maintaining its mechanical strength. It is also possible to make metallic foams that are not in the shape of a granule by this method. The major advantages of these granules are their low bulk density, high porosity, relatively high percentage of activated metal, relatively low production cost, and the activity these materials exhibit per kilogram of metal as well as the activity they have on a per liter of catalyst basis.

However the activated granules also have the same disadvantage as the tablets in that the expensive alloy inside the granule is not being used and if it is activated, it would be under mass transfer limitation and of lower value. Hence, it was important to develop a technology that activates and uses all of the rather expensive Raney-type alloy for the generation of a uniformly active phase on the catalyst.

Other supported Raney-type technologies include the creation of a thin Raney-type alloy layer on a support. This has been done by the rather expensive and somewhat awkward vacuum deposition of the metals in question onto the support to form a Raney-type alloy that in turn is activated with a caustic solution (DE19963443 A1). The cost of producing a commercial amount of supported Raney-type catalysts via the vacuum deposition of the metals in question would clearly be more expensive than the technology described by the invention of this patent and it would also be more difficult to produce a homogeneous product via vacuum deposition as well.

The production of a thin Raney-type alloy layer can also be achieved by the deposition of metallic salts onto the support, their rather expensive reduction and the caustic leaching of the resulting alloy. Not only would the quality of the catalyst be dependent on the degree of alloy formation during reduction, but the support to salt interaction will also play a role in the formation of the Raney-type alloy to be activated. Moreover, the complications involved in obtaining a uniform distribution of the metallic salts to be reduced to the alloy will lead to an inhomogeneous performance of the catalyst throughout the batch.

Thin supported Raney-type alloys have also be achieved by dipping a support into a molten NiAl alloy (Oden et al, U.S. Bur. Mines Rep. Invest., 1976,RI 8184, 13 pp. CODEN:XBM1A6) or by bringing an expensive catalytically active metal form into contact with molten Al or NiAl (US patent application 1976-707973). Not only does this harsh treatment during the formation of the alloy layer change the support, it also limits the types of supports that one can use for this application. It is clearly more difficult to reproduce the structure and performance of the corresponding Raney-type catalyst when the thin alloy layer was formed on a support, where cooling rates and metal interactions are very difficult to control, as opposed to a catalyst whose Raney-type alloy was produced under more controllable and cost-effective conditions and whose structure on the catalyst is produced in a very reproducible commercial scale.

Regardless of how one places a thin layer Raney-type alloy onto the surface of a support, the result will always be a catalyst with a low amount of active surface area in relation to the catalyst's total volume that is therefore more prone on a volumetric basis to deactivate and/or be poisoned during the reaction.

Another problem with Raney-type catalysts produced from thin alloy layers on supports is the lower mechanical strength of the active phase in which is more likely to sinter under milder conditions in comparison to typically Raney-type catalysts. These thin layer Raney-type supported catalysts also have all of their active sites on the outer most surface of the catalyst, where they are especially vunerable to abrasive forces that can remove the thin activated layer partially or totally.

Eggshell Raney-type catalyst technology has been further advanced by the development of activated hollow spheres (US Patents 6437186, 6486366 and 6437180 as well as European Patent 1068900). The activated hollow spheres have the advantage of a lower catalyst bulk density. The activated hollow spheres can be produced by spraying styrene balls with an alloy slurry, drying the coated spheres, burning out the styrofoam, calcining them (for improved mechanincal stability) and activating them in a aqueous caustic solution. The hollow spheres can also be produce by the coating of any evaporable or dissolvable carrier (e.g., polyethylene, various waxes and others) via different methods with the desired mixture of an alloy and/or metal together with organic and/or inorganic binders followed by the appropriate heat and/or dissolution treatment to remove the carrier. In the case of activated hollow spheres, the hollow alloy spheres remaining after removal of the carrier are then activated with an aqueous caustic solution. Other effective leaching reagents that remove the leachable component(s) of the alloy to form the catalyst may also be considered for the activation of this catalyst. Of course this technology can be applied to a wide range of hollow bodied objects and not only spheres. The disadvantage of this technology is the increased difficulty of production for the activated hollow spheres. The production of activated hollow spheres can be critical between the time the styrofoam carrier has been burnt out and the remaining metallic shell containing the alloy is stabilized. Although there has been improvements with the stabilization of the activated hollow spheres (W02005042153), the current technology of this invention is more robust and it allows for the higher production volumes and improved cost efficiencies required by today's industrial production of catalysts. An added benefit is the improved stability of the end catalyst made by the activation of eggshell alloy coated supports and the greater flexibility it offers for various reactor technology types.

Some fixed bed reactors have the reactants flow from the top to the bottom or the bottom to the top of the reactor where the reactants may be in a gas phase, a trickle phase, a liquid phase, combinations of these phases or diluted to some degree in any of these phases and their combinations with one or more solvents. In the case of hydrogenation, the reactants to be hydrogenated may flow co-current or counter-current to the direction of the hydrogen flow. In the case of oxidation, the reactants may also flow co- or counter-current to the direction of oxygen or any other type of oxidizing reagent. In some of these reactor configurations (e.g., upward reactant flow in a liquid phase as well as other configurations) there will be an increased amount of catalyst attrition and/or the decrease in catalyst performance via the development of preferred reactant flow channels due to the undesired movement of the catalyst in the reactor bed. This undesired catalyt movement during the reaction could be avoided by increasing the catalyst's density via the choice of support for the preparation of the catalyst as described by the invention of this patent.

In the case that catalyst movement in the reactor bed needs to be avoided, one would chose a support with a higher bulk density for the production of the catalyst of this invention. In the case where catalyst movement is not an issue, one may consider the use of lower density supports for the preparation of the catalyst of this patent, so as to improve some handling aspects of this material. Hence, the catalyst of this invention allows one to tailor-make a catalyst to fit the needs of the reactor technology to be employed.

An object of the present invention is therefore to provide "eggshell-type" fixed bed activated base metal catalysts where the expensive alloy and active sites are both located on the outer shell of the catalyst and as such, these catalysts largely avoid the disadvantages of the above known fixed-bed catalysts.

As dependent on the overall particle size of the coated Raney-type catalyst (e.g., powder supports), these catalysts may also be used as fluidized bed catalysts. Another application of the coated powderly supports would be their use in typical suspension phase reactions, such as the catalytic transformation of organic compounds as previously described.

The subject of the invention is a method of the production of supported metal catalysts, which is characterized in that
- the support is coated with an alloy containing substance, using one of the methods of the group rolling the support in an alloy containing substance, spraying the alloy containing substance onto the support; any other method that attaches the alloy particles on to the support or any combination of two or more of these methods

- the coated support is dried
- the coated support is calcined
- the coated support is activated

The method of the production of supported activated metal catalysis, according to the invention which can be characterized in that
- an alloy is dispersed in water
- optionally a metal powder is added to the dispersion
- optionally a pore builder is added to the dispersion
- the dispersion is sprayed on a support, in order to provide a first coating
- the coated support is dried
- the coated support is calcined
- the coated support is activated
- optionally coated the support is doped by coating the activated coated support with an aqueous solution of a metal-salt
- optionally the support is treated with an aqueous solution of LiOH

The method of the production of supported activated metal catalysis according to the invention can be characterized in that
- the dried coated support is sprayed with an aqueous dispersion to provide a second coating,
   whereby optionally the dispersion contains an alloy, whereby optionally the dispersion contains a pore builder,
   whereby optionally the dispersion contains a metal powder,
   whereby optionally the dispersion contains a metal oxide,
- then the for a second time coated support is dried
- then the for a second time coated support is calcined,
- then the for a second time coated support is activated,
- then optionally the activated coated support is treated with an aqueous solution of LiOH
- then the activated coated support is treated with an aqueous solution of a metal-salt.

The method of the production of supported activated metal catalysts according to the invention can be characterized in that, the dried coated support is sprayed with an aqueous dispersion to provide a second coating, whereby the alloy is the same alloy as used in the first coating.

The method of the production of supported activated metal catalysts according to the invention can be characterized in that, the dried coated support is sprayed with an aqueous dispersion containing an alloy to provide a second coating, whereby the alloy is a different alloy as used in the first coating.

The method of the production of supported activated metal catalysts according to the invention can be characterized in that, the dried coated support is sprayed with an aqueous dispersion containing a metal oxide to provide a second coating, whereby the metal oxide is one oxide selected from the group ZnO, Al₂O₃.

The method of the production of supported activated metal catalysis, according to the invention can be characterized in that the activated coated support is treated with an aqueous solution of a metal salt, whereby the metal salt is selected from the group of Fe, Pt, Mo or Pd.

The preparation of supported activated base metal catalysts can be done via the coating of a support with a mixture of the precursor alloy powder with optionally an organic binder, optionally one or more promoters (vida-infra) and optionally an inorganic binder to form a coated support that is dried, calcined for stabilization purposes and then activated in a caustic solution.

The precursor alloy can be either slowly cooled or rapidly cooled via contact, quenching, spraying in or spraying with a variety of mediums such as, but not limited to, inert gases and water. The precursor alloy can be comprised of a catalytic component, a caustic leachable component and optionally one or more promoters. The catalytic component can consist of one or more metals from groups VIII and Ib of the periodic chart of elements that are optionally promoted with one or more elements from the periodic groups Ia, Iia, IIIb, Ivb, Vb, Vib, VIIb, VIII, Ib, Iib, IIIa, Iva, Va and the rare earth elements. The caustic leachable component consists of Al, Si, Zn or mixtures therof.

The catalyst can be activated in a mixture of one or more metal hydroxides from the groups Ia and IIa of the periodic table.

The activated catalyst can be promoted via the adsorption of one or more elements from the periodic groups Ia, IIa, IIIb, IVb, Vb, VIb, VIIb, VIII, Ib, IIb, IIIa, Iva, Va and the rare earth elements onto the activated catalyst.

One or more promoters one or more elements from the periodic groups Ia, IIa, IIIb, IVb, Vb, VIb, VIIb, VIII, Ib, IIb, IIIa, Iva, Va and the rare earth elements can present in the alloy and the catalyst is further promoted after it has been activated in a basic solution via the adsorption of one or more elements from the periodic groups Ia, IIa, IIIb, IVb, Vb, VIb, VIIb, VIII, Ib, IIb, IIIa, Iva, Va and the rare earth elements.

The support can include carbon, alumina, carbon coated alumina, calcium carbonate, carbon black, Ion exchange materials, magnesia-alumina, niobia, silica, silica-alumina, magnesia, barium sulfate, titania, glasses, aluminum silicate, silica-titania, silicium carbide, zeolites, zirconium oxide, carbides, zinc oxide and mixtures thereof.

The alloy containing mixture can be sprayed onto the support as it is suspended in a fluidized fashion.

The alloy containing mixture can be sprayed onto the support as it is being mechanically mixed.

The supported activated base metal catalyst can be made by coating the support with various layers of different materials.

The supported activated base metal catalyst can be made by coating the support with various layers of different materials so that the outer layer acts as a poison trap that protects the inner catalytic layer.

These supported forms could either be filled into the reactor as is, they can be sintered together to form a desired structure, or they can be used in a fluidized bed as determined by the particle size of the supported Raney-Type catalyst.

The common forms include spheres, cylinders, ovals, tablets, rods, extrudated shapes and other forms as well. The main advantages of these catalysts are their relatively low production costs, highly adjustable bulk density, high stability and high porosity.

These catalysts are prepared by coating a support with a raney-type alloy *(vida-infra)* in such a way that only the outer shell of the catalyst contains the stabilized raney-type alloy. These materials are then converted into their corresponding supported activated base metal catalysts by their treatment with basic or acidic solutions (in some cases, other leaching reagents can be used to remove the alumina from the alloy in order to provide an active surface) to produce the hydrogen rich base metal skeletal structure via the leaching of the soluble component(s) incorporated into this raney-type alloy.

The coating on the support containing the Raney-type alloy may also contain pore builders, inorganic binders, organic binders, inert materials that may add texture and create a desired type of porosity, solid acids, solid bases and/or other materials that will enhance the properties of the invention.

The inorganic binders are typically metal powders, but they may also be other materials such as, but not limited to, ceramic and refractory materials.

The organic binders include, but are not limited to, preferably polyvinyl alcohols of the desired molecular weight and the potential pore builders include, but are not limited to, the materials named in patents US4826799 and US3351495. The pore builders can consist of waxes such as Licowax^{®} wax C micropowder, fats such as magnesium and aluminium stearates and carbohydrate containing polymers such as tylose (methyl celulose).

The Raney-type alloy coated support of this invention may also be made without the assistance of pore builders, intert material, inorganic binders and/or organic binders.

The Raney-type alloy coated support is optionally temperature treated or calcined so as to burn out any possibly present pore builders and binders and/or to sinter the binders or extremely small dendritic alloy phases that may also fuse the alloy particles together and act themselves as binders.

This catalyst precursor is then activated, as mentioned above, with bases preferably NaOH, KOH and/or there mixtures, acids and other leaching materials that will remove the soluble components (e.g., Al) from the alloy and leave behind an activated metal catalyst.

The coating of the support with the alloy can be performed by many different methods, such as, rolling the support in an alloy containing substance, spraying the alloy containing substance onto the support, the use of any other method that attaches the alloy particles on to the support or any combination of 2 or more of these methods. The best method is to spray a suspension of the alloy onto the support as it is either being mixed in a drum, conveyed on a belt, spun on a rotated disc, floated in a fluidized bed or by any other method that brings the alloy containing material (e.g., an alloy containing aqueous suspension) in contact with the support. It is preferred to spray the alloy suspension on to a fluidized bed of the support by spraying the alloy suspension from the top down onto the fluidized bed of support, spraying the alloy suspension from the bottom up to the fluidize bed of support, or to use a combination of both top and bottom spray either performed simultaneous, in series or in any combination thereof. Spraying the fluidized bed of support with the alloy suspension may also be done with the assistance of one or more cylinders, plates or any other directing devices in the spraying chamber so that the properties of the sprayed support can be best controlled. Another parameter that can be controlled for the further optimization of the sprayed eggshell catalysts' structure on the support is the fill level of the materials in the spraying chamber at the beginning and the end of the spraying.

One of the preferred methods involves spraying a bed of the support on a rotating shaped floor with the alloy suspension. The rotating shaped floor can be conical, shaped like a shallow bowl, shaped like a deep bowl or any other shape that directs the bed of support in the desired fashion. The rotating floor can also be perforated so that a gas can be forced through it to create different degrees of support fluidization above it. This rotating floor method (also known as the shovel rotor method) can also be used in combination with any sort of directing devices such as plates, cones, cylinders or any other suitable device in the spraying chamber so as to create the desired properties via the direction of the support, the spray or both. Other parts of the reactor may also rotate and/or be perforated either as a replacement to the rotating floor or in combination to the rotating floor. The alloy suspension may be either sprayed in the same direction as the support is rotated, in the opposite direction, in both directions at the same time or both in a alternating fashion of sometimes spraying with and sometimes against the direction of the support.

The alloy suspension may optionally contain organic and/or inorganic substances that may act as pore builders, binders and/or as modifiers for the eventual catalyst. The preferred suspension contains both an organic binder, such as but not limited to, polyvinyl alcohol and an inorganic binder, such as but not limited to, nickel powder. This suspension may also contain only an inorganic binder, only an organic binder or no binders at all.

The current invention covers the use of binders and any technology (i.e., the use of rapidly-cooled alloys with dendritic phase domains) that may not require binders for the stabilization of this catalyst.

The spraying of more than one materials onto support may be done at the same time where the mixture of the two or more components are kept constant or the amounts of each material may change as the outer shell is being built up. It is also possible to spray the inner layer with one material and the outer layer with another material and the number of layers of different materials or combinations of materials may be greater than one. Such changes in materials may include, but are not limited to, differences in alloy particle sizes, differences in alloy composition, differences in alloy structure, differences in alloy cooling rates (*vida-infra*), differences in material particle shapes, differences in binder combinations, the addition of one or more promoter elements, the addition of functionalized materials for the production of a multifunctional catalyst, the addition of poison traps and/or combinations of these as well as other materials. These parameters will allow one to change the properties of the resulting catalyst in such a way that the catalyst could be optimized for the application at hand.

Once the alloy suspension has been sprayed onto the support it may also be heat treated in any type of gas or gas combination that may enhance its stability or eventual performance of the resulting catalyst. This treatment gas may be either reducing (e.g., with hydrogen, mixtures containing hydrogen, hydrocarbons and/or mixtures thereof) The preferential treatment is performed in oxygen, air, synthetic air or other oxidizing agents and some of the positive effects of this gas treatment will include, but is not limited to, the sintering of the inorganic binder so as to build inorganic stabile bridges between the particles of the alloy, the burning out of pore builder(s), the sintering together of the smaller alloy phases within the same or between two or more different particles so as to stabilize their agglomerate, and/or the combination of these effects. The gas treatment temperature can range from room temperature to 1400°C or higher if done in such a fashion that produces a stabile catalyst precursor. This heat treatment may also be performed in a vacuum, especially if additional elements are to be vacuum deposited on to the catalyst precursor. Although it is not preferred, one may also form the alloy by vacuum depositing one or more of the elements of the alloy onto a support that has been coated with another component of the alloy. The heating of the coated support may be done with conventional equipment such as a muffle furnace, a belt furnace, a rotary calciner or any other piece of equipment similar in nature. The heat may be transferred via conduction through the wall of the heater, via the heating of the gases around the coated support, by the radiation of heat from a source flame, by the radiation from an infrared source, by the radiation from a microwaves source, by the radiation of the appropriate electromagnetic source and/or combinations thereof.

The technology of this invention allows one to form a stabilized outer shell of alloy particles onto a support of the desired density and potential functionality, where this alloy can be leached to form a very robust active phase that is mostly protected by the interparticle macroporosity of the catalytically active shell. The properties of the present invention can be influenced and adjusted by the choice of Raney-type alloy composition, Raney-type alloy particle size distribution, Raney-type alloy cooling rate, Raney-type alloy physical shape, the mixing of the Raney-type alloy with other materials, the use and choice of organic binders, the use and choice of pore builders, the choice of one or more catalytic metals in the Raney-type alloy, the choice of one or more soluble elements in the Raney-type alloy, the choice of the use and choice of one or more promoting elements introduced to the Raney-type alloy and/or to the activated catalyst, the use and choice of inorganic binders, the choice of support, the choice of coating method, the conditions of coating, and combinations of these properties. The catalytic metals include nickel, cobalt, copper, iron or mixtures thereof. are used as catalyst metals. Aluminum is generally used as the alloying component which is leachable (preferably under basic conditions with alkalis), but other components may also be used, in particular zinc and silicon or mixtures of these with aluminum.

These alloys may optionally contain one or more promoter elements before they are leached, or one or more promoters may be adsorbed onto the activated catalyst after it has been leached. In some cases it is also advantageous to have one or more promoters in the alloy prior to activation and to add one or more promoters to the activated promoted catalyst after it has been leached. In this situation, the one or more promoters that were added to the alloy may be the same or may be different than the one or more promoters that were added to the activated catalyst after leaching. Suitable promoter elements include those from the IA, IIA, IIIB, IVB, VB, VIB, VIIB, VIII, IB, IIB IIIA, IVA, VA, VIA, the Lanthanide and the actinide families of elements from the periodic table of elements.

The various materials and Raney-type alloys being supported onto the carrier can be done by the building of layers that may have well defined or graduate boundries between them in such a way as to optimize the catalyst for its use. One example would be to have a protective poison-trapping outer layer over a catalytic layer so as to improve the performance and lengthen the lifetime of the catalyst. Other factors known to those schooled in the art of catalysis can also be used with the current invention so as to improve the flexibility of the catalyst and the process that it is used in. The choice of supports for the present invention provides for catalysts of tuneable bulk densities and it makes it possible to create multifunctional catalysts where the support also participates in the reaction.

Furthermore, the technology of this invention affords one increase flexibility in the design of the catalyst, so that it can be tailor-made for a broader field of applications involving the catalytic transformations of organic molecules.

The above and other objects of the invention are achieved by coating a support having the desired properties with particles of the desired one or more Raney-type alloys optionally along with organic and/or inorganic binders as well as with pore builders, optionally drying these materials, optionally calcining these Raney-type alloy-coated support particles, and activating them in caustic solution in order to make them catalytic active.

Depending on the conditions of catalyst preparation, the desired catalyst properties, and the catalyst alloys being used, the use of organic and/or inorganic binders may or may not be necessary to obtain the appropriate supported Raney-type catalyst. The alloy-coated support can be made by bringing the support in contact with an alloy containing material in such a way that the alloy adheres to the support. This is preferably performed by spraying an alloy containing suspension onto the support, drying it and calcining it.

This alloy containing suspension can optionally contain the pore builders, binders, promoter elements, other materials with additional active sites (e.g., solid acids, solid bases and/or others) and inert materials that may provide the desired textural properties to the resulting catalytic layer. The organic binder can be chosen such that it expands or "foams up" during either mixing, drying or calcination thereby producing a metallic foam structure that exhibits a high porosity while maintaining its mechanical strength.

The major advantages of this invention are its high levels of flexibility that allow one to tailor-make the catalyst for the optimal performance of a reactor carrying out a reaction under the conditions of a commercially feasible process. This invention permits one to change the bulk density of the catalyst via the choice of support so as to improve handling and/or to avoid movement of the catalyst in the reactor bed that may cause mechanical attrition and reactant channeling. Not only does the catalyst of this invention have a readily adjustable bulk density, but the amount of expensive Raney-type alloy and corresponding active sites of the activated catalyst can also be readily controlled on both a weight and a volumetric basis leading to the more effective use of the catalyst.

The catalyst of this invention is especially most effective on a per weight basis of the catalytic metal, especially since all of the active sites are located at the utmost outer part of the catalyst thereby reducing any possible mass transfer limitations that may occur during the catalytical transformation of organic compounds.

The chosen supports may also be catalytically active and this would allow for the production of multifunctional catalysts that will enhance the selectivity and activity of the desired reaction. An example of this would be a reductive alkylation or reductive amination catalyst that uses an acidic support which would facilitate the condensation of an amine with a carbonyl compound to form the imine that is easily reduced by the supported Raney-type catalyst shell to the desired product.

The performance of these catalysts may be enhanced by the addition of one or more promoters either in the alloy before activation and/or by their adsorption onto the active catalyst after it has been leached with alkalis. Suitable promoters include metallic elements from the groups 1A, 2A, 3B, 4B, 5B, 6B, 7B, 8, 1B, 2B, 3A, 4A, 5A, 6A, and the rare earth elements of the periodic table.

Another aspect of this invention is the coating of the support with one type of powder and the ability to change the type of powder as the resulting coated support reaches a specific size to create a layered type of catalyst. This layering effect may be performed a number of times and this could be done continuously during the coating, or the coated support may be removed, dried and optionally calcined before it is added again to the coating apparatus for the addition of new powders. In these two ways, one could obtain coated supports with layering effects that have specific types of powders and/or various mixtures thereof at different depths of the granules. The support may also be coated with more than one powder suspension at the same time and the ratios of each powder suspension being sprayed onto the support can be optionally changed with respect to time so as to create another type of layering effect. The appropriate coating powders for this process include, but are not limited to, Raney-type catalytic metal / Al alloys, inert powders, catalytically active powders, promoters, poison traps, pore builders and binders.

The Raney-type catalytic metal / Al alloys may be slowly cooled or rapidly cooled alloys as described above, and its average particle size may range from ∼1 to 200 µm or more as required for the desired catalyst properties.

During spraying, the coated supports may be heated for the removal of the suspending liquid (e.g., water) so that the granules may proceed directly to calcination and/activation. The suspending liquid may also be left in the coated supports after spraying and in this case, it may be removed by drying before or during calcination. The suspending liquid may be left in the coated support or even increased so as to facilitate the formation of coated support clusters that provide structures that pack looser in a reactor and lead to lower pressure drops in comparison to other fixed bed geometries.

Perhaps the most important advantage of this technology is the tuneably low level or if needed, absence of unactivated alloy in the catalyst. The unactivated alloy in the center of especially activated chunk-types of catalyst may react uncontrollably under certain reaction conditions (e.g., sufficiently high temperatures in the presence of water, acidic compounds and basic compounds) to form aluminium based compounds and large volumes of hydrogen that may lead to an explosion and an undesired release of chemicals. In this respect, the catalyst of this invention will contain far less, if any at all, of the unactivated alloy thereby making this technology inherently safer than the previous ones where the levels of unactivated alloy in the catalyst are considerably higher.

The present invention will now be described in further detail. Supported Raney-type fixed bed catalyst precursors are formed by coating a typical support (carbon, alumina, carbon coated alumina, calcium carbonate, carbon black, Ion exchange materials, magnesia-alumina, niobia, silica, silica-alumina, magnesia, barium sulfate, titania, glasses, aluminum silicate, silica-titania, silicium carbide, zeolites, zirconium oxide, carbides, zinc oxide, mixtures of the aforementioned supports and others as well) with one or more Raney-type alloy(s). One could also coat a mixture of support types and structures. The current patent covers all the possible ways one can produce such a material, however the preferred method is to spray a liquid suspension containing the one or more desired alloys, optionally an organic binder (e.g., polyvinyl alcohol), water, optionally an inorganic binder (e.g., Ni, Co, Fe, Cu, other metal powders, refractive materials, ceramic materials and others), optionally promoters, and optionally pore builders onto the support of choice. The one or more supports can be coated in an impregnation drum, in a spraying chamber where the one or more supports are kept suspended as a fluidized bed and/or by any other coating procedure. The resulting coated support particles are then optionally dried and calcined to the desired temperature (e.g. from 100 to 1200°C), activated with an alkali leaching solution (e.g. an aqueous caustic solution), and washed with basic (e.g., caustic) and/or pH neutral aqueous solutions for the production of this fixed bed activated base metal catalyst. The choice of an organic binder, the types of other components, the ratio of the various components and the treatment of the resulting supported Raney-type catalyst may be done so that the resulting alloy coating of the support foams up and creates a metallic foam structure that has a high porosity and lower bulk density. This higher porosity can lead to a more complete leaching process and it leads to a catalyst with a higher activity for catalytic chemical reactions. However the production of the "nonfoamed" type of alloy coating structure is also covered by this invention and it is even preferred by some reactions and reactor types due to its increased mechanical stability.

The bulk density of the resulting fixed bed catalyst is very important for highly active catalysts and this technology allows one to adjust the density of the catalyst to the fit the reactor technology inwhich it will be used. The ratio by weight of catalyst metal to extractable alloying component in the catalyst alloy is, as is conventional with Raney alloys, preferably in the range from 20:80 to 80:20. Catalysts according to the invention may also be doped with other metals in order to have a positive effect on the catalytic properties of the invention. The purpose of this type of doping is, for example, to improve the activity, selectivity, and lifetime of the catalyst in a specific reaction. Doping metals are frequently also called promoters. The doping or promoting of Raney catalyst is described for example in U.S. patent 4,153, 578 and DE-AS 21 01 856 in DE-OS 21 00 373 and in the DE-AS 2053799.

In principle, any known metal alloys with extractable elements such as aluminum zinc and Silicon maybe used for the present invention. Suitable promoters are transition elements in groups of 3B to 7B and 8 and group 1B of the Periodic Table of Elements and also the rare-earth metals. The other elements mentioned previously in this document are also considered to be suitable promoters. They are also used in an amount of up to 20 wt% or more, with respect of the total weight of catalyst. Chromium, manganese, iron, cobalt, vanadium, tantalum, titanium, cerium tungsten, rhenium, platinum, palladium, ruthenium, nickle, copper, silver, gold, and/or molybdenum and metals from platinum group are preferably used as promoters. The promoting elements could be added as alloying constituents in the catalyst alloy and/or they could be added to the catalyst after activation. In addition, promoters may be added as binders, they may be present as separate alloy powders with extractable elements and/or, they may be added to the catalyst after calcination and before activation. Optimum adjustment of the catalyst properties to the particular catalyst process is thus possible.

The Raney-type coated support catalyst precursors resulting from calcination are also very important with regard the economic viability of this invention and are also convered by it. They are not pyrophoric and can be handled and transported without difficulty. Activation can be performed by the user shortly before use. Storage under water or organic solvents or embedding in organic compounds is not required for the catalyst precursors.

The catalyst according to the invention can be used for the transformation of organic compounds, for the hydrogenation of organic compounds, for the partial hydrogenation of organic compounds, for the hydrogenation of carbonyl groups in organic compounds, for the hydrogenation of acetone, for the hydrogenation of aldehydes, for the hydrogenation of ketones, for the hydrogenation of glucose, for the hydrogenation of sugars to polyols, for the hydrogenation of aldoses to polyols, for the hydrogenation of ketoses to polyols, for the hydrogenation of monosacharide aldoses to polyols, for the hydrogenation of monosacharide ketoses to polyols, for the hydrogenation of disacharide aldoses to polyols, for the hydrogenation of disacharide ketoses to polyols, for the hydrogenation of multisacharide aldoses to polyols, for the hydrogenation of multisacharide ketoses to polyols, for the hydrogenation of nitriles, for the hydrogenation of imines, for the hydrogenation of dinitriles, for the hydrogenation of adiponitrile, for the hydrogenation of alkene moeities in organic compounds, for the hydrogenation of alkyne moeities in organic compounds, for the hydrogenation of aromatics, for the hydrogenation of 2-butyne-1,4-diol, for the hydrogenation of nitro groups in organic compounds, for the hydrogenation of dinitro compounds, for the hydrogenation of aromatic nitrocompounds, for the hydrogenation of aromatic dinitrocompounds, for the hydrogenation of dinitrotoluene.

Another part of this invention is the use of these supported activated metal catalysts for catalytic chemical reactions such as the hydrogenation, isomerization, hydration, hydrogenolysis, reductive amination, reductive alklyation, dehydration, oxidation, dehydrogenation, rearrangement and other catalytic transformation of organic compounds. These catalysts are preferred for the continuous hydrogenation of organic compounds such as nitro compounds, nitriles, imines, carbonyl compounds, alkenes, alkynes and aromatic compounds. These moeities may have already existed in the reactant(s) or they may have been incorporated into the reactant(s) just prior or during the reaction. These reactants may be sugars, nitriles, dinitriles, nitro compound, dinitro compounds and multifunctional compounds. These catalysts may also be used for the removal of some groups, such as, protecting groups used for multi-step organic synthesis, halides and sulfur containing compounds (e.g., thiols) via their hydrogenolysis.

### Examples

### Example 1:

### Silica supported activated Ni / Al catalyst from a casted Ni / Al alloy having an activated shell thickness of 100 µm

A coating solution was prepared by suspending 532 grams of a 53%Ni/47%Al casted alloy and 43 grams of a pure nickel powder (99%Ni with a d₅₀ of 21 µm) into an aqueous solution containing 518 grams of water and 10.4 grams of polyvinylalcohol (PVA). This suspension was then sprayed onto 1000 ml of Poraver SiO₂-glass spheres with a bulk density of 230 g/l ranging from 1 to 2 mm in diameter while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). The dried coated SiO₂-glass spheres were then calcined in air at 750°C in order to burn out the PVA and to sinter the Ni and alloy particles together thereby forming a stabilized outer shell. This calcined catalyst precursor had a bulk density of 494 grams per liter. An amount of 100 grams of these calcined alloy coated SiO₂-glass catalyst precursors were activated with an aqueous 20 wt.% NaOH solution for 1.5 hours at 90°C, washed in a light caustic solution and washed in water to produce catalyst E1. Another 494 grams of these calcined alloy coated SiO₂-glass catalyst precursors were used to make the catalyst in example 2.

### Example 2:

### Silica supported activated Ni / Al catalyst from a casted Ni / Al alloy having an activated shell thickness of 200 µm.

A coating solution was prepared by suspending 462 grams of a 53%Ni/47%Al casted alloy and 37 grams of a pure nickel powder (99%Ni with a d₅₀ of 21 µm) into an aqueous solution containing 450 grams of water and 9.0 grams of polyvinylalcohol (PVA). This suspension was then sprayed onto 1000 ml of the calcined alloy coated Poraver SiO₂-glass spheres from example 1 having a bulk density of 494 g/l while they were suspended in an upwardly flowing air stream (inert gases such as nitrogen can also be used). After coating the coated SiO₂-glass spheres with the above mentioned solution, they were then dried in an upwardly flowing stream of air (inert gases such as nitrogen can also be used) at temperatures up to 80°C (higher or lower temperatures may also be used). The dried coated SiO₂-glass spheres were then calcined in air at 750°C in order to burn out the PVA and to sinter the Ni and alloy particles together thereby forming a stabilized outer shell. This calcined catalyst precursor had a bulk density of 658 grams per liter. An amount of 100 grams of these calcined alloy coated SiO₂-glass catalyst precursors were activated with an aqueous 20 wt.% NaOH solution for 1.5 hours at 90°C and washed in water to produce catalyst E2. Another 658 grams of these calcined alloy coated SiO₂-glass catalyst precursors were used to make the catalyst in example 3.

### Example 3:

### Silica supported activated Ni / Al catalyst from a casted Ni / Al alloy having an activated shell thickness of 300 µm.

A coating solution was prepared by suspending 409 grams of a 53%Ni/47%Al casted alloy and 33 grams of a pure nickel powder (99%Ni with a d₅₀ of 21 µm) into an aqueous solution containing 398 grams of water and 8.0 grams of polyvinylalcohol (PVA). This suspension was then sprayed onto 1000 ml of the calcined alloy coated Poraver SiO₂-glass spheres from example 2 having a bulk density of 658 g/l while they were suspended in an upwardly flowing air stream (inert gases such as nitrogen can also be used). After coating the coated SiO₂-glass spheres with the above mentioned solution, they were then dried in an upwardly flowing stream of air (inert gases such as nitrogen can also be used) at temperatures up to 80°C (higher or lower temperatures may also be used). The dried coated SiO₂-glass spheres were then calcined in air at 750°C in order to burn out the PVA and to sinter the Ni and alloy particles together thereby forming a stabilized outer shell. This calcined catalyst precursor had a bulk density of 1033,8 grams per liter. An amount of 100 grams of these calcined alloy coated SiO₂-glass catalyst precursors were activated with an aqueous 20 wt.% NaOH solution for 1.5 hours at 90°C, washed in a light caustic solution and washed in water to produce catalyst E3. Another 766 grams of these calcined alloy coated SiO₂-glass catalyst precursors were used to make the catalyst in example 4.

### Example 4:

### Silica supported activated Ni / Al catalyst from a casted Ni / Al alloy having an activated shell thickness of 400 µm.

A coating solution was prepared by suspending 366 grams of a 53%Ni/47%Al casted alloy and 30 grams of a pure nickel powder (99%Ni with a d₅₀ of 21 µm) into an aqueous solution containing 356 grams of water and 7.1 grams of polyvinylalcohol (PVA). This suspension was then sprayed onto 1000 ml of the calcined alloy coated Poraver SiO₂-glass spheres from example 3 having a bulk density of 766 g/l while they were suspended in an upwardly flowing air stream (inert gases such as nitrogen can also be used). After coating the coated SiO₂-glass spheres with the above mentioned solution, they were then dried in an upwardly flowing stream of air (inert gases such as nitrogen can also be used) at temperatures up to 80°C (higher or lower temperatures may also be used). The dried coated SiO₂-glass spheres were then calcined in air at 750°C in order to burn out the PVA and to sinter the Ni and alloy particles together thereby forming a stabilized outer shell. This calcined catalyst precursor had a bulk density of 1102,3 grams per liter. An amount of 100 grams of these calcined alloy coated SiO₂-glass catalyst precursors were activated with an aqueous 20 wt.% NaOH solution for 1.5 hours at 90°C, washed in a light caustic solution and washed in water to produce catalyst E4.

### Example 5:

### Magnesia-Silica supported activated Ni / Al catalyst from a casted Ni / Al alloy having an activated shell thickness of 100 µm.

A coating solution was prepared by suspending 249 grams of a 53%Ni/47%Al casted alloy and 20 grams of a pure nickel powder (99%Ni with a d₅₀ of 21 µm) into an aqueous solution containing 486 grams of water and 9.7 grams of polyvinylalcohol (PVA). This suspension was then sprayed onto 1000 ml of 3 mm Ceramtec MgO-SiO₂ spheres with a bulk density of 1300 g/l while they were suspended in an upwardly flowing air stream (inert gases such as nitrogen can also be used). After coating the MgO-SiO₂ spheres with the above mentioned solution, the spheres were then dried in an upwardly flowing stream of air (inert gases such as nitrogen can also be used) at temperatures up to 80°C (higher or lower temperatures may also be used). The dried coated MgO-SiO₂ spheres were then calcined in air at 750°C in order to burn out the PVA and to sinter the Ni and alloy particles together thereby forming a stabilized outer shell. This calcined catalyst precursor had a bulk density of 1260 grams per liter. An amount of 200 grams of these calcined alloy coated MgO-SiO₂ catalyst precursors were activated with an aqueous 20 wt.% NaOH solution for 1.5 hours at 90°C, washed in a light caustic solution and washed in water to produce catalyst E5. Another 494 grams of these calcined alloy coated SiO₂-glass catalyst precursors were used to make the catalyst in example 6.

### Example 6:

### Magnesia-Silica supported activated Ni / Al catalyst from a casted Ni / Al alloy having an activated shell thickness of 260 µm.

A coating solution was prepared by suspending 386 grams of a 53%Ni/47%Al casted alloy and 31 grams of a pure nickel powder (99%Ni with a d₅₀ of 21 µm) into an aqueous solution containing 752 grams of water and 15.0 grams of polyvinylalcohol (PVA). This suspension was then sprayed onto 1000 ml of the calcined alloy coated Ceramtec MgO-SiO₂ spheres from example 5 having a bulk density of 1260 g/l while they were suspended in an upwardly flowing air stream (inert gases such as nitrogen can also be used). After coating the coated MgO-SiO₂ spheres with the above mentioned solution, they were then dried in an upwardly flowing stream of air (inert gases such as nitrogen can also be used) at temperatures up to 80°C (higher or lower temperatures may also be used). The dried coated MgO-SiO₂ spheres were then calcined in air at 750°C in order to burn out the PVA and to sinter the Ni and alloy particles together thereby forming a stabilized outer shell. This calcined catalyst precursor had a bulk density of 1213 grams per liter. An amount of 200 grams of these calcined alloy coated MgO-SiO₂ sphere catalyst precursors were activated with an aqueous 20 wt.% NaOH solution for 1.5 hours at 90°C and washed in water to produce catalyst E6.

### Example 7:

### Silica supported activated Co / Al catalyst from a casted Co / Al alloy with a diameter of 3400 µm and an activated shell thickness of 200 µm.

A coating solution was prepared by suspending 776 grams of a 50%Co/50%Al casted alloy into an aqueous solution containing 699 grams of water and 14.0 grams of polyvinylalcohol (PVA). This suspension was then sprayed onto 1350 ml of 3 mm Poraver SiO₂-glass spheres with a bulk density of 189 g/l while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). These dried uncalcined coated SiO₂-glass spheres had a bulk density of 466 grams per liter. An amount of 1350 ml of the dried uncalcined coated SiO₂-glass spheres were put aside for the preparation of catalyst E8 and the rest of them were then calcined in air at 750°C in order to burn out the PVA and to sinter the alloy particles together thereby forming a stabilized outer shell. These calcined alloy coated SiO₂-glass catalyst precursors were activated with an aqueous 20 wt.% NaOH solution for 1.5 hours at 90°C, washed in a light caustic solution and washed in water to produce catalyst E7.

### Example 8:

### Silica supported activated Co / Al catalyst from a casted Co / Al alloy with a diameter of 3800 µm and an activated shell thickness of 400 µm.

A coating solution was prepared by suspending 675 grams of a 50%Co/50%Al casted alloy into an aqueous solution containing 607 grams of water and 12.1 grams of polyvinylalcohol (PVA). This suspension was then sprayed onto 1350 ml of the dried uncalcined alloy coated Poraver SiO₂-glass spheres from example 7 having a bulk density of 466 g/l while they were suspended in an upwardly flowing air stream (inert gases such as nitrogen can also be used). After coating the coated SiO₂-glass spheres with the above mentioned solution, they were then dried in an upwardly flowing stream of air (inert gases such as nitrogen can also be used) at temperatures up to 80°C (higher or lower temperatures may also be used). These dried uncalcined coated SiO₂-glass spheres had a bulk density of 638 grams per liter. An amount of 1350 ml of the dried uncalcined coated SiO₂-glass spheres were put aside for the preparation of catalyst E9 and the rest of them were then calcined in air at 750°C in order to burn out the PVA and to sinter the alloy particles together thereby forming a stabilized outer shell. These calcined alloy coated SiO₂-glass catalyst precursors were activated with an aqueous 20 wt.% NaOH solution for 1.5 hours at 90°C and washed in water to produce catalyst E8.

### Example 9:

### Silica supported activated Co / Al catalyst from a casted Co / Al alloy with a diameter of 4200 µm and an activated shell thickness of 600 µm.

A coating solution was prepared by suspending 597 grams of a 50%Co/50%Al casted alloy into an aqueous solution containing 537 grams of water and 10.7 grams of polyvinylalcohol (PVA). This suspension was then sprayed onto 1350 ml of the dried uncalcined alloy coated Poraver SiO₂-glass spheres from example 8 having a bulk density of 638 g/l while they were suspended in an upwardly flowing air stream (inert gases such as nitrogen can also be used). After coating the coated SiO₂-glass spheres with the above mentioned solution, they were then dried in an upwardly flowing stream of air (inert gases such as nitrogen can also be used) at temperatures up to 80°C (higher or lower temperatures may also be used). These dried uncalcined coated SiO₂-glass spheres had a bulk density of 751 grams per liter. The dried coated SiO₂-glass spheres were then calcined in air at 750°C in order to burn out the PVA and to sinter the alloy particles together thereby forming a stabilized outer shell. These calcined alloy coated SiO₂-glass catalyst precursors were activated with an aqueous 20 wt.% NaOH solution for 1.5 hours at 90°C, washed in a light caustic solution and washed in water to produce catalyst E9.

### Example 10:

### Silica supported activated Co / Al catalyst from a casted Co / Al alloy with diameters ranging from 1400 to 2400 µm and an activated shell thickness of 200 µm.

A coating solution was prepared by suspending 1759 grams of a 50%Co/50%Al casted alloy into an aqueous solution containing 1583 grams of water and 31.7 grams of polyvinylalcohol (PVA). This suspension was then sprayed onto 1350 ml of Poraver SiO₂-glass spheres with a bulk density of 230 g/l ranging from 1 to 2 mm in diameter while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). These dried uncalcined coated SiO₂-glass spheres had a bulk density of 658 grams per liter. An amount of 1350 ml of the dried uncalcined coated SiO₂-glass spheres were put aside for the preparation of catalyst E11 and the rest of them were then calcined in air at 750°C in order to burn out the PVA and to sinter the alloy particles together thereby forming a stabilized outer shell. These calcined alloy coated SiO₂-glass catalyst precursors were activated with an aqueous 20 wt.% NaOH solution for 1.5 hours at 90°C, washed in a light caustic solution and washed in water to produce catalyst E10.

### Example 11:

### Silica supported activated Co / Al catalyst from a casted Co / Al alloy with diameters ranging from 1800 to 2800 µm and an activated shell thickness of 400 µm.

A coating solution was prepared by suspending 1319 grams of a 50%Co/50%Al casted alloy into an aqueous solution containing 1187 grams of water and 23.7 grams of polyvinylalcohol (PVA). This suspension was then sprayed onto 1350 ml of the dried uncalcined alloy coated Poraver SiO₂-glass spheres from example 10 having a bulk density of 658 g/l while they were suspended in an upwardly flowing air stream (inert gases such as nitrogen can also be used). After coating the coated SiO₂-glass spheres with the above mentioned solution, they were then dried in an upwardly flowing stream of air (inert gases such as nitrogen can also be used) at temperatures up to 80°C (higher or lower temperatures may also be used). These dried uncalcined coated SiO₂-glass spheres had a bulk density of 839 grams per liter. An amount of 1350 ml of the dried uncalcined coated SiO₂-glass spheres were put aside for the preparation of catalyst E12 and the rest of them were then calcined in air at 750°C in order to burn out the PVA and to sinter the alloy particles together thereby forming a stabilized outer shell. These calcined alloy coated SiO₂-glass catalyst precursors were activated with an aqueous 20 wt.% NaOH solution for 1.5 hours at 90°C, washed in a light caustic solution and washed in water to produce catalyst E11.

### Example 12:

### Silica supported activated Co / Al catalyst from a casted Co / Al alloy with diameters ranging from 2200 to 3200 µm and an activated shell thickness of 600 µm.

A coating solution was prepared by suspending 1053 grams of a 50%Co/50%Al casted alloy into an aqueous solution containing 947 grams of water and 18.9 grams of polyvinylalcohol (PVA). This suspension was then sprayed onto 1350 ml of the dried uncalcined alloy coated Poraver SiO₂-glass spheres from example 11 having a bulk density of 839 g/l while they were suspended in an upwardly flowing air stream (inert gases such as nitrogen can also be used). After coating the coated SiO₂-glass spheres with the above mentioned solution, they were then dried in an upwardly flowing stream of air (inert gases such as nitrogen can also be used) at temperatures up to 80°C (higher or lower temperatures may also be used). These dried uncalcined coated SiO₂-glass spheres had a bulk density of 928 grams per liter. The dried coated SiO₂-glass spheres were then calcined in air at 750°C in order to burn out the PVA and to sinter the alloy particles together thereby forming a stabilized outer shell. These calcined alloy coated SiO₂-glass catalyst precursors were activated with an aqueous 20 wt.% NaOH solution for 1.5 hours at 90°C, washed in a light caustic solution and washed in water to produce catalyst E12.

### Example 13:

### LiOH treated Silica supported activated Co / Al catalyst from a casted Co / Al alloy with a diameter of 3800 µm and an activated shell thickness of 400 µm.

An amount of 100 grams of the E8 catalyst was treated for 3 hours in a 10%LiOH solution followed by being washed with water to produce catalyst E13.

### Example 14:

### Silica supported activated Ni / Al catalyst from a Ni / Al alloy that was sprayed cooled in nitrogen.

A coating solution was prepared by suspending 780 grams of a 40%Ni/60%Al alloy that was sprayed cooled in nitrogen into an aqueous solution containing 700 grams of water and 14.0 grams of polyvinylalcohol (PVA). This suspension was then sprayed onto 1350 ml of 3 mm Poraver SiO₂-glass spheres with a bulk density of 189 g/l while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). The dried uncalcined coated SiO₂-glass spheres were removed from the spraying chamber and of the approximately 2 liters, only 1350 ml of them were placed back into the spraying vessel for a second coating.

The second coating solution was prepared by suspending 680 grams of a 40%Ni/60%Al alloy that was sprayed cooled in nitrogen into an aqueous solution containing 610 grams of water and 12 grams of polyvinylalcohol (PVA). This suspension was then sprayed onto the previously coated 1350 ml of 3 mm Poraver SiO₂-glass spheres while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). These twice coated dried SiO₂-glass spheres were then calcined in air at 750°C in order to burn out the PVA and to sinter the alloy particles together thereby forming a stabilized outer shell. These calcined alloy coated SiO₂-glass catalyst precursors were activated with an aqueous 20 wt.% NaOH solution for 1.5 hours at 90°C, washed in a light caustic solution and washed in water to produce catalyst E14.

### Example 15:

### Silica supported activated Ni / Al catalyst from a Ni / Al alloy that was sprayed cooled in water.

A coating solution was prepared by suspending 63 grams of a pure Ni powder (99%Ni with a d₅₀ of 21 µm) and 780 grams of a 50%Ni/50%Al alloy that was sprayed cooled in water into an aqueous solution containing 700 grams of water and 14.0 grams of polyvinylalcohol (PVA). This suspension was then sprayed onto 1350 ml of 3 mm Poraver SiO₂-glass spheres with a bulk density of 189 g/l while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). The dried uncalcined coated SiO₂-glass spheres were removed from the spraying chamber and of the approximately 2 liters, only 1350 ml of them were placed back into the spraying vessel for a second coating.

The second coating solution was prepared by suspending 55 grams of a pure Ni powder (99%Ni with a d₅₀ of 21 µm) and 680 grams of a 40%Ni/60%Al alloy that was sprayed cooled in water into an aqueous solution containing 610 grams of water and 12 grams of polyvinylalcohol (PVA). This suspension was then sprayed onto the previously coated 1350 ml of 3 mm Poraver SiO₂-glass spheres while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). These twice coated dried SiO₂-glass spheres were then calcined in air at 750°C in order to burn out the PVA and to sinter the alloy particles together thereby forming a stabilized outer shell. These calcined alloy coated SiO₂-glass catalyst precursors were activated with an aqueous 20 wt.% NaOH solution for 1.5 hours at 90°C, washed in a light caustic solution and washed in water to produce catalyst E15.

### Example 16:

### Silica supported activated Cu / Al catalyst from a casted Cu / Al alloy.

A coating solution was prepared by suspending 63 grams of a pure Ni powder (99%Ni with a d₅₀ of 21 µm) and 780 grams of a casted 50%Cu/50%Al alloy into an aqueous solution containing 700 grams of water and 14.0 grams of polyvinylalcohol (PVA). This suspension was then sprayed onto 1350 ml of 3 mm Poraver SiO₂-glass spheres with a bulk density of 189 g/l while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). The dried uncalcined coated SiO₂-glass spheres were removed from the spraying chamber and of the approximately 2 liters, only 1350 ml of them were placed back into the spraying vessel for a second coating.

The second coating solution was prepared by suspending 55 grams of a pure Ni powder (99%Ni with a d₅₀ of 21 µm) and 680 grams of a casted 50%Cu/50%Al alloy into an aqueous solution containing 610 grams of water and 12 grams of polyvinylalcohol (PVA). This suspension was then sprayed onto the previously coated 1350 ml of 3 mm Poraver SiO₂-glass spheres while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). These twice coated dried SiO₂-glass spheres were then calcined in air at 750°C in order to burn out the PVA and to sinter the alloy particles together thereby forming a stabilized outer shell. These calcined alloy coated SiO₂-glass catalyst precursors were activated with an aqueous 20 wt.% NaOH solution for 1.5 hours at 90°C, washed in a light caustic solution and washed in water to produce catalyst E16.

### Example 17:

### Pt doped Silica supported activated Cu / Al catalyst from a casted Cu / Al alloy.

An amount of 100 grams of the E16 catalyst was treated in a circulating aqueous solution containing hexachloroplatinic acid, whose pH value was adjusted upto a pH of 7 with NaOH prior to the addition of the catalyst. The Pt-doped catalyst was then washed and the final platinum content of the catalyst (E17) was 0.3%Pt.

### Example 18:

### Fe doped Silica supported activated Cu / Al catalyst from a casted Cu / Al alloy.

An amount of 100 grams of the E16 catalyst was treated in a circulating aqueous solution containing iron(III)chloride, whose pH value was adjusted upto a pH of 7 with NaOH prior to the addition of the catalyst. The Fe-doped catalyst was then washed and the final platinum content of the catalyst (E18) was 1.0%Fe.

### Example 19:

### Silica supported activated Cu / Zn / Al catalyst from a Cu / Zn / Al alloy that was spray cooled in water.

A coating solution was prepared by suspending 63 grams of a pure Ni powder (99%Ni with a d₅₀ of 21 µm) and 780 grams of a 50%Cu / 35%Zn / 15%Al alloy that was spray cooled in water into an aqueous solution containing 700 grams of water and 14.0 grams of polyvinylalcohol (PVA). This suspension was then sprayed onto 1350 ml of 3 mm Poraver SiO₂-glass spheres with a bulk density of 189 g/l while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). The dried uncalcined coated SiO₂-glass spheres were removed from the spraying chamber and of the approximately 2 liters, only 1350 ml of them were placed back into the spraying vessel for a second coating.

The second coating solution was prepared by suspending 55 grams of a pure Ni powder (99%Ni with a d₅₀ of 21 µm) and 680 grams of a 50%Cu / 35%Zn / 15%Al alloy that was spray cooled in water into an aqueous solution containing 610 grams of water and 12 grams of polyvinylalcohol (PVA).

This suspension was then sprayed onto the previously coated 1350 ml of 3 mm Poraver SiO₂-glass spheres while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). These twice coated dried SiO₂-glass spheres were then calcined in air at 750°C in order to burn out the PVA and to sinter the alloy particles together thereby forming a stabilized outer shell. These calcined alloy coated SiO₂-glass catalyst precursors were activated with an aqueous 20 wt.% NaOH solution for 1.5 hours at 90°C, washed in a light caustic solution and washed in water to produce catalyst E19.

### Example 20:

### Silica supported activated Ni / Mo / Al catalyst from a Ni / Mo / Al alloy that was spray cooled in nitrogen.

A coating solution was prepared by suspending 63 grams of a pure Ni powder (99%Ni with a d₅₀ of 21 µm) and 780 grams of a 39%Ni / 1%Mo / 60%Al alloy that was spray cooled in nitrogen into an aqueous solution containing 700 grams of water and 14.0 grams of polyvinylalcohol (PVA). This suspension was then sprayed onto 1350 ml of 3 mm Poraver SiO₂-glass spheres with a bulk density of 189 g/l while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). The dried uncalcined coated SiO₂-glass spheres were removed from the spraying chamber and of the approximately 2 liters, only 1350 ml of them were placed back into the spraying vessel for a second coating.

The second coating solution was prepared by suspending 55 grams of a pure Ni powder (99%Ni with a d₅₀ of 21 µm) and 680 grams of a 39%Ni / 1%Mo / 60%Al alloy that was spray cooled in nitrogen into an aqueous solution containing 610 grams of water and 12 grams of polyvinylalcohol (PVA). This suspension was then sprayed onto the previously coated 1350 ml of 3 mm Poraver SiO₂-glass spheres while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). These twice coated dried SiO₂-glass spheres were then calcined in air at 750°C in order to burn out the PVA and to sinter the alloy particles together thereby forming a stabilized outer shell. These calcined alloy coated SiO₂-glass catalyst precursors were activated with an aqueous 20 wt.% NaOH solution for 1.5 hours at 90°C, washed in a light caustic solution and washed in water to produce catalyst E20.

### Example 21:

### Silica supported activated Co / Cr / Al catalyst from a Co / Cr / Al alloy that was spray cooled in water.

A coating solution was prepared by suspending 780 grams of a 30%Co / 1%Cr / 59%Al alloy that was spray cooled in water into an aqueous solution containing 700 grams of water and 14.0 grams of polyvinylalcohol (PVA). This suspension was then sprayed onto 1350 ml of 3 mm Poraver SiO₂-glass spheres with a bulk density of 189 g/l while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). The dried uncalcined coated SiO₂-glass spheres were removed from the spraying chamber and of the approximately 2 liters, only 1350 ml of them were placed back into the spraying vessel for a second coating.

The second coating solution was prepared by suspending 680 grams of a 30%Co / 1%Cr / 59%Al alloy that was spray cooled in water into an aqueous solution containing 610 grams of water and 12 grams of polyvinylalcohol (PVA). This suspension was then sprayed onto the previously coated 1350 ml of 3 mm Poraver SiO₂-glass spheres while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). These twice coated dried SiO₂-glass spheres were then calcined in air at 750°C in order to burn out the PVA and to sinter the alloy particles together thereby forming a stabilized outer shell. These calcined alloy coated SiO₂-glass catalyst precursors were activated with an aqueous 20 wt.% NaOH solution for 1.5 hours at 90°C, washed in a light caustic solution and washed in water to produce catalyst E21.

### Example 22:

### LiOH treated Silica supported activated Co / Cr / Al catalyst from a Co / Cr / Al alloy that was spray cooled in water.

An amount of 100 grams of the E21 catalyst was treated for 3 hours in a 10%LiOH solution followed by being washed with water to produce catalyst E22.

### Example 23:

### Silica supported activated Ni / Cr / Fe / Al catalyst from a Ni / Cr / Fe / Al alloy that was spray cooled in water.

A coating solution was prepared by suspending 63 grams of a pure Ni powder (99%Ni with a d₅₀ of 21 µm) and 780 grams of a 40%Ni / 1%Cr / 0.5%Fe / 58.5%Al alloy that was spray cooled in water into an aqueous solution containing 700 grams of water and 14.0 grams of polyvinylalcohol (PVA). This suspension was then sprayed onto 1350 ml of 3 mm Poraver SiO₂-glass spheres with a bulk density of 189 g/l while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). The dried uncalcined coated SiO₂-glass spheres were removed from the spraying chamber and of the approximately 2 liters, only 1350 ml of them were placed back into the spraying vessel for a second coating.

The second coating solution was prepared by suspending 55 grams of a pure Ni powder (99%Ni with a d₅₀ of 21 µm) and 680 grams of a 40%Ni / 1%Cr / 0.5%Fe / 58.5%Al alloy that was spray cooled in water into an aqueous solution containing 610 grams of water and 12 grams of polyvinylalcohol (PVA). This suspension was then sprayed onto the previously coated 1350 ml of 3 mm Poraver SiO₂-glass spheres while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). These twice coated dried SiO₂-glass spheres were then calcined in air at 750°C in order to burn out the PVA and to sinter the alloy particles together thereby forming a stabilized outer shell. These calcined alloy coated SiO₂-glass catalyst precursors were activated with an aqueous 20 wt.% NaOH solution for 1.5 hours at 90°C, washed in a light caustic solution and washed in water to produce catalyst E23.

### Example 24

### Pd doped silica supported activated Ni / Cr / Fe / Al catalyst from a Ni / Cr / Fe / Al alloy that was spray cooled in water.

An amount of 100 grams of the E23 catalyst was treated for 6 hours in a circulating aqueous solution containing palladium nitrate solution that previously had its pH adjusted to 6 with sodium carbonate. The end Pd content of the catalyst E24 was 0.2%.

### Example 25:

### Mo doped silica supported activated Ni / Al catalyst from a casted Ni / Al alloy.

An amount of 60 grams of the E4 catalyst was treated for 6 hours in a circulating aqueous solution containing sodium molybdate and after this post activation doping, the molybdenum content of catalyst E25 was 0.3%. The procedure can also be carried out with other Mo containing compounds such as ammonium heptamolybdate.

### Example 26:

### Alumina supported activated Ni / Al catalyst from a casted Ni / Al alloy.

A coating solution was prepared by suspending 63 grams of a pure Ni powder (99%Ni with a d₅₀ of 21 µm) and 780 grams of a casted 53%Ni / 47%Al alloy into an aqueous solution containing 700 grams of water and 14.0 grams of polyvinylalcohol (PVA). This suspension was then sprayed onto 1350 ml of ∼3.2 mm Alcoa CSS-350 alumina spheres with a bulk density of 740 g/l while they were suspended in an upwardly flowing air stream (inert gases such as nitrogen can also be used). After coating the alumina spheres with the above mentioned solution, the spheres were then dried in an upwardly flowing stream of air (inert gases such as nitrogen can also be used) at temperatures up to 80°C (higher or lower temperatures may also be used). The dried uncalcined coated alumina spheres were removed from the spraying chamber and of the approximately 2 liters, only 1350 ml of them were placed back into the spraying vessel for a second coating.

The second coating solution was prepared by suspending 55 grams of a pure Ni powder (99%Ni with a d₅₀ of 21 µm) and 680 grams of a casted 53%Ni / 47%Al alloy into an aqueous solution containing 610 grams of water and 12 grams of polyvinylalcohol (PVA). This suspension was then sprayed onto the previously coated 1350 ml of 3 mm ∼3.2 mm Alcoa CSS-350 alumina spheres while they were suspended in an upwardly flowing air stream (inert gases such as nitrogen can also be used). After coating the alumina spheres with the above mentioned solution, the spheres were then dried in an upwardly flowing stream of air (inert gases such as nitrogen can also be used) at temperatures up to 80°C (higher or lower temperatures may also be used). These twice coated dried alumina spheres were then calcined in air at 750°C in order to burn out the PVA and to sinter the alloy particles together thereby forming a stabilized outer shell. These calcined alloy coated SiO₂-glass catalyst precursors were activated with an aqueous 20 wt.% NaOH solution for 1.5 hours at 90°C, washed in a light caustic solution and washed in water to produce catalyst E26.

### Example 27:

### Titania supported activated Ni / Al catalyst from a casted Ni / Al alloy.

A coating solution was prepared by suspending 63 grams of a pure Ni powder (99%Ni with a d₅₀ of 21 µm) and 780 grams of a casted 53%Ni / 47%Al alloy into an aqueous solution containing 700 grams of water and 14.0 grams of polyvinylalcohol (PVA). This suspension was then sprayed onto 1350 ml of 5 mm P-25 titania tablets with a bulk density of 1270 g/l while they were suspended in an upwardly flowing air stream (inert gases such as nitrogen can also be used). After coating the titania tablets with the above mentioned solution, the tablets were then dried in an upwardly flowing stream of air (inert gases such as nitrogen can also be used) at temperatures up to 80°C (higher or lower temperatures may also be used). The dried uncalcined coated titania tablets were removed from the spraying chamber and of the approximately 2 liters, only 1350 ml of them were placed back into the spraying vessel for a second coating.

The second coating solution was prepared by suspending 55 grams of a pure Ni powder (99%Ni with a d₅₀ of 21 µm) and 680 grams of a casted 53%Ni / 47%Al alloy into an aqueous solution containing 610 grams of water and 12 grams of polyvinylalcohol (PVA). This suspension was then sprayed onto the previously coated 1350 ml of 5 mm P-25 titania tablets while they were suspended in an upwardly flowing air stream (inert gases such as nitrogen can also be used). After coating the titania tablets with the above mentioned solution, the tablets were then dried in an upwardly flowing stream of air (inert gases such as nitrogen can also be used) at temperatures up to 80°C (higher or lower temperatures may also be used). These twice coated dried titania tablets were then calcined in air at 750°C in order to burn out the PVA and to sinter the alloy particles together thereby forming a stabilized outer shell. These calcined alloy coated titania catalyst precursors were activated with an aqueous 20 wt.% NaOH solution for 1.5 hours at 90°C, washed in a light caustic solution and washed in water to produce catalyst E27.

### Example 28:

### Silica supported activated metal catalyst made with an inner layer of activated nickel that is protected with an outer layer of activated Ag.

A coating solution was prepared by suspending 63 grams of a pure Ni powder (99%Ni with a d₅₀ of 21 µm) and 780 grams of a casted 53%Ni / 47%Al alloy into an aqueous solution containing 700 grams of water and 14.0 grams of polyvinylalcohol (PVA). This suspension was then sprayed onto 1350 ml of 3 mm Poraver SiO₂-glass spheres with a bulk density of 189 g/l while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). The dried uncalcined coated SiO₂-glass spheres were removed from the spraying chamber and of the approximately 2 liters, only 1350 ml of them were placed back into the spraying vessel for a second coating.

The second coating solution was prepared by suspending 55 grams of a pure Ni powder (99%Ni with a d₅₀ of 21 µm) and 680 grams of a casted 50%Ag / 50%Al alloy into an aqueous solution containing 610 grams of water and 12 grams of polyvinylalcohol (PVA). This suspension was then sprayed onto the previously coated 1350 ml of 3 mm Poraver SiO₂-glass spheres while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). These twice coated dried SiO₂-glass spheres were then calcined in air at 750°C in order to burn out the PVA and to sinter the alloy particles together thereby forming a stabilized outer shell. These calcined alloy coated SiO₂-glass catalyst precursors were activated with an aqueous 20 wt.% NaOH solution for 1.5 hours at 90°C, washed in a light caustic solution and washed in water to produce catalyst E28. The outer activated Ag layer acts as a trap for certain poisons (halides and certain sulfur species) and allows the activated Ni layer below it to function as a catalyst for a longer time without being exposed to the above mentioned catalyst deactivating agents. The thickness of the outer protective activated Ag layer can be adjusted to optimize its effectiveness.

### Example 29:

### Silica supported activated metal catalyst made with an inner layer of activated nickel that is protected with an outer layer of zinc oxide.

A coating solution was prepared by suspending 63 grams of a pure Ni powder (99%Ni with a d₅₀ of 21 µm) and 780 grams of a casted 53%Ni / 47%Al alloy into an aqueous solution containing 700 grams of water and 14.0 grams of polyvinylalcohol (PVA). This suspension was then sprayed onto 1350 ml of 3 mm Poraver SiO₂-glass spheres with a bulk density of 189 g/l while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). The dried uncalcined coated SiO₂-glass spheres were removed from the spraying chamber and of the approximately 2 liters, only 1350 ml of them were placed back into the spraying vessel for a second coating.

The second coating solution was prepared by suspending 680 grams of a zinc oxide powder into an aqueous solution containing 610 grams of water and 12 grams of polyvinylalcohol (PVA). This suspension was then sprayed onto the previously coated 1350 ml of 3 mm Poraver SiO₂-glass spheres while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). These twice coated dried SiO₂-glass spheres were then calcined in air at 750°C in order to burn out the PVA and to sinter the alloy particles together thereby forming a stabilized outer shell. These calcined alloy coated SiO₂-glass catalyst precursors were activated with an aqueous 20 wt.% NaOH solution for 1.5 hours at 90°C, washed in a light caustic solution and washed in water to produce catalyst E29. The outer zinc oxide layer acts as a trap for certain poisons (e.g., certain sulfur species) and allows the activated Ni layer below it to function as a catalyst for a longer time without being exposed to the above mentioned catalyst deactivating agents. The thickness of the outer protective zinc oxide layer can be adjusted to optimize its effectiveness.

### Example 30:

### Silica supported activated metal catalyst made with an inner layer of activated nickel that is protected with an outer layer of aluminum oxide.

A coating solution was prepared by suspending 63 grams of a pure Ni powder (99%Ni with a d₅₀ of 21 µm) and 780 grams of a casted 53%Ni / 47%Al alloy into an aqueous solution containing 700 grams of water and 14.0 grams of polyvinylalcohol (PVA). This suspension was then sprayed onto 1350 ml of 3 mm Poraver SiO₂-glass spheres with a bulk density of 189 g/l while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). The dried uncalcined coated SiO₂-glass spheres were removed from the spraying chamber and of the approximately 2 liters, only 1350 ml of them were placed back into the spraying vessel for a second coating.

The second coating solution was prepared by suspending 680 grams of a aluminum oxide powder into an aqueous solution containing 610 grams of water and 12 grams of polyvinylalcohol (PVA). This suspension was then sprayed onto the previously coated 1350 ml of 3 mm Poraver SiO₂-glass spheres while they were suspended in an upwardly flowing air stream (inert gases such as nitrogen can also be used). After coating the SiO₂-glass spheres with the above mentioned solution, the spheres were then dried in an upwardly flowing stream of air (inert gases such as nitrogen can also be used) at temperatures up to 80°C (higher or lower temperatures may also be used). These twice coated dried SiO₂-glass spheres were then calcined in air at 750°C in order to burn out the PVA and to sinter the alloy particles together thereby forming a stabilized outer shell. These calcined alloy coated SiO₂-glass catalyst precursors were activated with an aqueous 20 wt.-% NaOH solution for 1.5 hours at 90°C, washed in a light caustic solution and washed in water to produce catalyst E30. The outer aluminium oxide layer acts as a trap for certain poisons (e.g., certain phosphate species) and allows the activated Ni layer below it to function as a catalyst for a longer time without being exposed to the above mentioned catalyst deactivating agents. The thickness of the outer protective aluminium oxide layer can be adjusted to optimize its effectiveness.

### Comparative examples

### Comparative example 1:

### Shell activated Ni / Al tablets from a rapidly cooled water sprayed Ni / Al alloy.

In accordance with the literature (US Patents 5536694 and 6262307), the preparation of the Ni / Al shell activated tablets started with a homogeneous mixture of 1000 grams of a 50%Ni / 50%Al rapidly cooled water sprayed alloy, 75 grams of a pure Ni powder (99%Ni and D₅₀ = 21 µm) and 50 grams of ethylene bis-stearylamide that was tableted to 3 by 3 mm tablets, calcined for 2 hours at 700°C, activated in a 20 wt.-% caustic solution for 2 hours at 80°C, washed in a caustic solution, washed in water and stored under a mildly caustic aqueous solution (pH ∼10.5) until use. The bulk densities of four different batches of catalyst CE1 were 1.56, 1.58, 1.75 and 1.79 grams per mL.

### Comparative Example 2:

### Shell activated Cu / Al tablets from a rapidly cooled water sprayed Cu / Al alloy

In accordance with the literature (US Patents 5536694 and 6262307), the preparation of the Cu / Al shell activated tablets started with a homogeneous mixture of 1000 grams of a 50%Cu / 50%Al rapidly cooled water sprayed alloy, 75 grams of a pure Ni powder (99%Ni and D₅₀ = 21 µm) and 50 grams of ethylene bis-stearylamide that was tableted to 3 by 3 mm tablets, calcined for 2 hours at 700°C, activated in a 20 wt.-% caustic solution for 2 hours at 80°C, washed in a caustic solution, washed in water and stored under a mildly caustic aqueous solution (pH ∼10.5) until use. The bulk density of catalyst CE2 was 1.76 grams per mL.

### Comparative Example 3:

### Shell activated Co / Al tablets from a slowly cooled casted Co / Al alloy

In accordance with the literature (US Patents 5536694 and 6262307), the preparation of the Co / Al shell activated tablets started with a homogeneous mixture of 1000 grams of a 50%Co / 50%Al slowly cooled casted alloy and 50 grams of ethylene bis-stearylamide that was tableted to 3 by 3 mm tablets, calcined for 2 hours at 700°C, activated in a 20 wt.% caustic solution for 2 hours at 80°C, washed in a caustic solution, washed in water and stored under a mildly caustic aqueous solution (pH ∼10.5) until use. The bulk density of catalyst CE2 was 2.06 grams per mL.

### Application Example 1:

### The hydrogenation of acetone.

The trickle phase hydrogenation of acetone was carried out over the activated base metal catalysts in a tube reactor in the presence of hydrogen at 75°C and 5 bar. The data from these tests are displayed in table 1.

**Table 1**

| *The acetone hydrogenation data.* | | | | | |
|---|---|---|---|---|---|
| Catalyst | LHSV | %Con.^{A} | IPA %Sel.^{B} | Activity per gram^{C} | Activity per ml^{D} |
| E4 | 3.40 | 93.3 | 100 | 80,2 | 43.7 |
| CE1 | 3.43 | 56.3 | 100 | 16.9 | 26.3 |
| E16 | 3.05 | 40.1 | 99.2 | 41.9 | 16.6 |
| E18 | 3.06 | 91.9 | 99.8 | 95.4 | 38.2 |
| CE2 | 3.09 | 27.6 | 99.3 | 6.6 | 11.6 |

| | | | | | |
|---|---|---|---|---|---|
| A. The percentage of acetone conversion during the reaction. B. The percent isopropanol selectivity. C. The mmoles of acetone reacted per gram of catalyst per hour. D. The mmoles of acetone reacted per ml of catalyst per hour. | | | | | |

### Application Example 2:

### The hydrogenation of glucose.

The trickle phase hydrogenation of glucose was carried out over an activated base metal catalyst in a tube reactor in the presence of hydrogen with a 40% aqueous glucose solution at 140°C and 50 bar. The data from this test are displayed in table 2.

**Table 2:**

| *The glucose hydrogenation data.* | | | | |
|---|---|---|---|---|
| Catalyst | LHSV | %Con.^{B} | Activity per gram^{C} | Activity per ml^{D} |
| E25 | 3 | 33.8 | 1.42 | 2.60 |

| | | | | |
|---|---|---|---|---|
| A. The percentage of glucose conversion during the reaction. B. The mmoles of glucose reacted per gram of catalyst per hour. C. The mmoles of glucose reacted per ml of catalyst per hour. | | | | |

### Application Example 3:

### The hydrogenation of 2-butyne-1,4-diol.

The trickle phase hydrogenation of 2-butyne-1,4-diol was carried out over the activated base metal catalysts in a tube reactor at 135°C and 60 bar in the presence of hydrogen with a 50% aqueous 2-butyne-1,4-diol solution that was adjusted to a pH of 7 with NaHCO₃. The data from these tests are displayed in table 3.

**Table 3:**

| The 2-butyne-1,4-diol hydrogenation data. | | | | | | | |
|---|---|---|---|---|---|---|---|
| Catalyst | Throughput^{A} | %Con.^{B} | BDO %Sel^{C} | BeD %Sel^{D} | BDO / BeD Ratio^{E} | Activity per gram^{F} | Activity per ml^{G} |
| E15 | 1.60 | 76.5 | 36.3 | 39.0 | 0.93 | 2,89 | 1.23 |
| E20 | 1.60 | 93.1 | 48.5 | 30.1 | 1.61 | 4.14 | 1.50 |
| CE1 | 1.60 | 62.8 | 28.6 | 49.3 | 0.58 | 0.574 | 1.01 |

| | | | | | | | |
|---|---|---|---|---|---|---|---|
| A. The throughput of 2-butyne-1,4-diol in grams per ml of catalyst per hour. B. The percentage of 2-butyne-1,4-diol conversion during the reaction. C. The percent 1,4-butanediol selectivity. D. The percent 2-butene-1,4-diol selectivity. E. The 1,4-butanediol to 2-butene-1,4-diol ratio. F. The mmoles of 2-butyne-1,4-diol reacted per gram of catalyst per hour. G. The mmoles of 2-butyne-1,4-diol reacted per ml of catalyst per hour. | | | | | | | |

### Application Example 4:

### The hydrogenation of adiponitrile via method 1.

The trickle phase hydrogenation of adiponitrile was carried out over the activated base metal catalysts in a tube reactor at 65 bar in the presence of hydrogen with a 20% adiponitrile in methanol solution. The data from these tests are displayed in table 4.

**Table 4:**

| *The adiponitrile hydrogenation data via method 1.* | | | | | | | | |
|---|---|---|---|---|---|---|---|---|
| Catalyst | Time h | Temp. °C | LHSV | HMD %Sel^{A} | ACN %Sel^{B} | %Con^{C} | Activity per gram^{D} | Activity per ml^{E} |
| E8 | 4.35 | 110 | 1 | 65.1 | 17.6 | 84.94 | 27.96 | 7.62 |
| E15 | 4.5 | 110 | 1 | 57.3 | 8.08 | 95.29 | 33.11 | 8.48 |
| | 6.2 | 110 | 0.25 | 59.4 | 1.10 | 100 | 8.83 | 2.26 |
| E23 | 4.4 | 117 | 1 | 43.2 | 17.8 | 90.58 | 39.86 | 8.17 |
| | 6.9 | 110 | 0.25 | 44.6 | 11.1 | 100 | 10.92 | 2.24 |
| E13 | 4, 1 | 110 | 1 | 70.5 | 21.1 | 84.67 | 27.42 | 7.47 |
| CE1 | 2.80 | 152.2 | 0.25 | 17.90 | 14.90 | 93.30 | 1.19 | 2.13 |
| | 4.65 | 153.9 | 0.51 | 29.64 | 10.57 | 96.50 | 2.48 | 4.43 |
| CE3 | 2.70 | 112 | 1.03 | 46.93 | 43.59 | 41.98 | 1.88 | 3.86 |
| | 3.97 | 111 | 0.26 | 70.97 | 13.61 | 86.50 | 0.97 | 2.00 |

| | | | | | | | | |
|---|---|---|---|---|---|---|---|---|
| A. The percent hexamethylendiamine selectivity. B. The percent aminocapronitrile selectivity. C. The percentage of adiponitrile conversion during the reaction. D. The mmoles of adiponitrile reacted per gram of catalyst per hour. E. The mmoles of adiponitrile reacted per ml of catalyst per hour. | | | | | | | | |

### Application Example 5:

### The hydrogenation of adiponitrile via method 2.

The trickle phase hydrogenation of adiponitrile was carried out over the activated base metal catalysts in a tube reactor at 65 bar in the presence of hydrogen with a 20% adiponitrile in methanol solution, where one liter of this methanol originally contained 1.9 grams of NaOH. The data from this test is displayed in table 5.

**Table 5:**

| *The adiponitrile hydrogenation data via method 2.* | | | | | | | | |
|---|---|---|---|---|---|---|---|---|
| Catalyst | Time h | Temp. °C | LHSV | HMD %Sel^{A} | ACN %Sel^{B} | %Con^{C} | Activity per gram^{D} | Activity per ml^{E} |
| E23 | 4.3 | 110 | 1 | 72.90 | 25.89 | 92.99 | 40.95 | 8.39 |
| | 5.1 | 110.5 | 1 | 79.20 | 20.1 | 97.47 | 42.59 | 8.73 |

| | | | | | | | | |
|---|---|---|---|---|---|---|---|---|
| A. The percent hexamethylendiamine selectivity. B. The percent aminocapronitrile selectivity. C. The percentage of adiponitrile conversion during the reaction. D. The mmoles of adiponitrile reacted per gram of catalyst per hour. E. The mmoles of adiponitrile reacted per ml of catalyst per hour. | | | | | | | | |

### Application Example 6:

### The hydrogenation of adiponitrile via method 3.

The trickle phase hydrogenation of adiponitrile was carried out over the activated base metal catalysts in a tube reactor at 65 bar in the presence of hydrogen with a 20% adiponitrile in methanol solution, where one liter of this methanol originally contained 1.9 grams of LiOH. The data from this test is displayed in table 6.

**Table 6:**

| *The adiponitrile hydrogenation data via method 3.* | | | | | | | | |
|---|---|---|---|---|---|---|---|---|
| Catalyst | Time h | Temp. °C | LHSV | HMD %Sel^{A} | ACN %Sel^{B} | %Con^{C} | Activity per gram^{D} | Activity per ml^{E} |
| E8 | 4.05 | 110 | 1 | 86.41 | 11.82 | 92.74 | 28.68 | 7.82 |

| | | | | | | | | |
|---|---|---|---|---|---|---|---|---|
| A. The percent hexamethylendiamine selectivity. B. The percent aminocapronitrile selectivity. C. The percentage of adiponitrile conversion during the reaction. D. The mmoles of adiponitrile reacted per gram of catalyst per hour. E. The mmoles of adiponitrile reacted per ml of catalyst per hour. | | | | | | | | |

### Application Example 7:

### The hydrogenation of dinitrotoluene.

The trickle phase hydrogenation of dinitrotoluene was carried out over the activated base metal catalysts in a tube reactor at ∼80°C and 60 bar in the presence of hydrogen with a 4% dinitrotoluene in methanol solution. The data from these tests are displayed in table 7.

**Table 7 :**

| *The dinitrotoluene hydrogenation data.* | | | | | | |
|---|---|---|---|---|---|---|
| Catalyst | Temp.°C | Throughput^{A} | TDA %Sel^{B} | %Con^{C} | Activity per gram^{D} | Activity per ml^{E} |
| E23 | 81 | 0.31 | 99.2 | 98.6 | 9.01 | 1.847 |
| E24 | 81 | 0.31 | 99.8 | 100 | 9.28 | 1.902 |
| CE1 | 84.2 | 0.310 | 95.016 | 92.32 | 0.994 | 1.573 |

| | | | | | | |
|---|---|---|---|---|---|---|
| A. The dinitrotoluene throughput in grams of dinitrotoluene per ml of catalyst per hour. B. The percent toluenediamine selectivity. C. The percentage of dinitrotoluene conversion during the reaction. D. The mmoles of dinitrotoluene reacted per gram of catalyst per hour. E. The mmoles of dinitrotoluene reacted per ml of catalyst per hour. | | | | | | |

## Claims

1. Method of the production of supported metal catalysts, **characterized in that**
- the support is coated with an alloy containing substance,
- using at least one of the methods of the group
(a) rolling the support in an alloy containing substance,
(b) spraying the alloy containing substance onto the support,
(c) any other method that attaches the alloy particles on to the support or any combination of two or more of these methods
- the coated support is dried
- the coated support is calcined
- the coated support is activated

2. A method of the production of supported activated metal catalysis according to claim 1, **characterized in that**
- an alloy is dispersed in water
- optionally a metal powder is added to the dispersion
- optionally a pore builder is added to the dispersion
- the dispersion is sprayed on a support, in order to provide a first coating
- the coated support is dried
- the coated support is calcined
- the coated support is activated
- optionally the support is doped by coating the activated coated support with an aqueous solution of a metal-salt
- optionally the support is treated with an aqueous solution of LiOH.

3. A method of the production of supported activated metal catalysis according to claim 1 or 2, **characterized in that**
- the dried support is sprayed with an aqueous dispersion to provide a second coating,
whereby optionally the dispersion contains an alloy, whereby optionally the dispersion contains a pore builder,
whereby optionally the dispersion contains a metal powder,
whereby optionally the dispersion contains a metal oxide,
- then the for a second time coated support is dried
- then the for a second time coated support is calcined,
- then the for a second time coated support is activated,
- then optionally the activated coated support is treated with an aqueous solution of LiOH
- then the activated coated support is treated with an aqueous solution of a metal-salt.

4. Method of the production of supported activated metal catalysts according to claim 2,
**characterized in that**,
the dried coated support is sprayed with an aqueous dispersion to provide a second coating, whereby the alloy is the same alloy as used in the first coating.

5. Method of the production of supported activated metal catalysts according to claim 2,
**characterized in that**,
the dried coated support is sprayed with an aqueous dispersion containing an alloy to provide a second coating, whereby the alloy is a different alloy as used in the first coating.

6. Method of the production of supported activated metal catalysts according to claim 2,
**characterized in that**,
the dried coated support is sprayed with an aqueous dispersion containing a metal oxide to provide a second coating, whereby the metal oxide is one oxide selected from the group ZnO, Al₂O₃.

7. Method of the production of supported activated metal catalysis, according to claim 1 or 2,
**characterized in that**,
the activated coated support is treated with an aqueous solution of a metal salt, whereby the metal salt is selected from the group of Fe, Pt, Mo or Pd.

8. Use of the supported activated metal catalysis for organic transformation, i.e. the hydrogenation of organic compounds.
